# EUROPEAN PATENT APPLICATION

(11) **EP 4 620 492 A1**
(43) Date of publication of application: **24.09.2025**
(21) Application number: 23892033.4
(22) Date of filing: 16.11.2023
(51) Int. Cl.: A61L 27/18, A61L 27/58, C08L 71/02, C08L 67/04

(54) **POWDER FORMULATION FOR TISSUE REPAIR, AND INJECTABLE COMPOSITION FOR TISSUE REPAIR COMPRISING SAME**

(30) Priority: 17.11.2022 KR 20220154884
(71) Applicant: Samyang Holdings Corporation, Seoul 03129 (KR)
(72) Inventor: PARK, Ji Hoon, Suwon-si Gyeonggi-do 16509 (KR); KO, Suk Yen, Suwon-si Gyeonggi-do 16505 (KR); PARK, Na Jeong, Yongin-si Gyeonggi-do 16852 (KR); YOON, Hye Sung, Seongnam-si Gyeonggi-do 13544 (KR); LEE, Jong Pil, Seoul 05668 (KR)
(74) Representative: Gleiss Große Schrell und Partner mbB
(86) International application number: PCT/KR2023/018442
(87) International publication number: WO 2024/106970

(57) **Abstract**

The present invention relates to a powder formulation for tissue repair and an injectable composition for tissue repair comprising same and, more particularly, to: a powder formulation comprising a copolymer of a hydrophilic biocompatible polymer and a hydrophobic biocompatible polymer, wherein when a reference solution comprising the powder formulation at a specific concentration is diluted according to a series of increasing dilution factors, the number of polymer particles in the diluted solution is greater than or equal to the number of polymer particles in the reference solution at a particular dilution factor interval, and then exhibits a decreasing behavior as the dilution factors increase; and a tissue repair injectable composition comprising same.

## Description

### TECHNICAL FIELD

The present invention relates to a powder formulation for tissue repair treatment and an injection composition for tissue repair treatment comprising the same, and more specifically, a powder formulation comprising a copolymer of hydrophilic biocompatible polymer and hydrophobic biocompatible polymer, and an injection composition for tissue repair treatment comprising the same, wherein when a reference solution containing the powder formulation at a specific concentration is diluted according to a series of subsequently increasing dilution factors, the number of polymer particles in diluted solution is the same as or higher than the number of polymer particles in the reference solution within a specific dilution factor range, and then shows a decreasing behavior as the dilution factor increases.

### BACKGROUND ART

Recently, many people are interested in well-aging, which is growing old gracefully from a young age. It is no exaggeration to say that the current beauty market is focusing on anti-aging, which is aging slowly, beautifully and healthily. One of the most common signs of aging is a decrease in volume. In particular, since no volume in the face can make older and shabby look, people are very interested in fillers that can provide volume. Accordingly, the filler market is growing rapidly every year, and currently forms a market worth more than 2 trillion won worldwide.

Various substances are currently used as filler materials, and among them hyaluronic acid fillers occupy more than 90% of the global filler market, but have problems that the half-life in the body is within 1 to 3 days and so the persistence is very low and the resorption in the body occurs very rapidly. Thus, products having a resorption period extended by connecting hyaluronic acid with crosslinking material are on market. However, in case of such crosslinked products, because the crosslinking material, BDDE (1,4-butanediol diglycidyl ether), is a toxic carcinogen, there are problems that process cost increases according to removal process thereof, and loss is caused by product disposal due to microbial contamination, product disposal due to detection of residues, etc.

Accordingly, many tissue repair products using polymers that decompose in vivo have been developed, and as filler formulations using conventional biocompatible polymers, formulations have been developed by processing water-insoluble polymers into micro-sized particles and then dispersing them through a viscous excipient or thickener, and used. For example, a formulation in which poly(lactic acid) (PLA) particles with a diameter of 20 to 50 micrometers are dispersed in an aqueous solution of carboxymethylcellulose (CMC) or a formulation in which poly(caprolactone) (PCL) particles with a diameter of 20 to 50 micrometers are dispersed in an aqueous solution of CMC and glycerin have been used, but such formulations have problems of inconvenience in surgery due to needle clogging by the microparticles during injection and failure to obtain uniform tissue repair effect due to not uniformly dispersed particles.

### CONTENTS OF THE INVENTION

### PROBLEMS TO BE SOLVED

The purpose of the present invention is to provide a tissue repair product which has no risk of toxicity, secures functionality, properties, and safety suitable for biomaterials for tissue repair, and exhibits excellent tissue repair effects while being easily transported, stored, and handled, and a method for preparing the same.

### TECHNICAL MEANS

In an aspect, the present invention provides a powder formulation for tissue repair treatment, comprising a copolymer of hydrophilic biocompatible polymer and hydrophobic biocompatible polymer, wherein:
when a reference solution containing the powder formulation at a concentration of 7.5 % by weight is diluted according to a series of subsequently increasing dilution factors,
in a dilution factor range of from 4 to 8, the number of polymer particles per unit volume in diluted solution is the same as or higher than the number of polymer particles per unit volume in the reference solution; and
in a dilution factor range of 16 or higher, the number of polymer particles per unit volume in diluted solution decreases as the dilution factor increases.

In other aspect, the present invention provides an injection composition for tissue repair treatment, comprising the powder formulation for tissue repair treatment of the present invention; and a pharmaceutically acceptable carrier for injection.

In another aspect, the present invention provides a method for preparing an injection composition for tissue repair treatment, the method comprising: mixing the powder formulation for tissue repair treatment of the present invention and a pharmaceutically acceptable carrier for injection at room temperature.

### EFFECT OF THE INVENTION

The powder formulation for tissue repair treatment of the present invention has no toxicity and is easily transported, stored and handled, and forms polymer particles which are easily dispersed and dissolved in aqueous medium at room temperature, and after being introduced into the body, it is not phagocytosed by macrophages and induces collagen, showing an excellent tissue repair effect.

### BRIEF EXPLANATION OF THE DRAWINGS

Figure 1 shows the results of measuring the number of particles of Preparation Examples 1 to 4 conducted in Experimental Example 2 of the present invention.
Figure 2 shows the results of measuring the number of particles of Comparative Examples 1 to 5 conducted in Experimental Example 2 of the present invention.
Figure 3 shows the results of animal tests for tissue repair effect conducted in Experimental Example 3 of the present invention.

### CONCRETE MODE FOR CARRYING OUT THE INVENTION

The present invention will be explained in detail below.

The powder formulation for tissue repair treatment of the present invention comprises a copolymer of hydrophilic biocompatible polymer and hydrophobic biocompatible polymer.

The powder formulation for tissue repair treatment of the present invention is characterized in that, when a reference solution containing the powder formulation at a concentration of 7.5 % by weight is diluted according to a series of subsequently increasing dilution factors, (i) in a dilution factor range of from 4 to 8, the number of polymer particles per unit volume in diluted solution is the same as or higher than the number of polymer particles per unit volume in the reference solution; and (ii) in a dilution factor range of 16 or higher, the number of polymer particles per unit volume in diluted solution decreases as the dilution factor increases.

In an embodiment, a medium used for preparation of the reference solution and dilution thereof may be an aqueous medium, for example, phosphate buffer, water for injection, saline, distilled water, purified water, or glucose solution.

In an embodiment, the series of subsequently increasing dilution factors may include dilution factors of 2, 4, 8, 16, 32 and 64, but it is not limited thereto.

As used herein, "dilution factor" means, when diluting the reference solution, the volume multiple of the diluted solution compared to the volume of the reference solution. For example, "a diluted solution with dilution factor of 4" means a diluted solution prepared by adding a medium equivalent to 3 times the volume of the reference solution into the standard solution so that the final volume is 4 times the volume of the reference solution.

As used herein, "dilution factor range" is determined according to the dilution factors applied when diluting the reference solution. For example, diluted solutions with dilution factors of at least 4 and up to 8 are included in "a dilution factor range of from 4 to 8," and diluted solutions with dilution factors of 16 or higher are included in "a dilution factor range of 16 or higher."

As used herein, "in a certain dilution factor range, the number of polymer particles per unit volume in diluted solution decreases as the dilution factor increases" means that the number of polymer particles per unit volume in a solution with a dilution factor in the dilution factor range is higher than the number of polymer particles per unit volume in a solution with a higher dilution factor. In the present invention, in a dilution factor range of 16 or higher, the number of polymer particles per unit volume in diluted solution decreases as the dilution factor increases, and thus, for example, the number of polymer particles per unit volume in a solution with dilution factor of 16 is higher than the number of polymer particles per unit volume in a solution with dilution factor of 32.

In an embodiment, the powder formulation for tissue repair treatment of the present invention may be characterized in that, in a dilution factor range of from 4 to 16, the number of polymer particles per unit volume in diluted solution is the same as or higher than the number of polymer particles per unit volume in the reference solution.

In an embodiment, the powder formulation for tissue repair treatment of the present invention may be characterized in that, in a dilution factor range of from 4 to 32, the number of polymer particles per unit volume in diluted solution is the same as or higher than the number of polymer particles per unit volume in the reference solution.

In an embodiment, the powder formulation for tissue repair treatment of the present invention may be characterized in that, in a dilution factor range of 8 or higher, the number of polymer particles per unit volume in diluted solution decreases as the dilution factor increases.

In an embodiment, the powder formulation for tissue repair treatment of the present invention may be characterized in that, in a dilution factor range of 4 or higher, the number of polymer particles per unit volume in diluted solution decreases as the dilution factor increases.

In an embodiment, the powder formulation for tissue repair treatment of the present invention may be characterized in that, at dilution factor of 32, the number of polymer particles per unit volume in diluted solution is 60 % or higher (for example, 60 to 99 %), based on 100 % of the number of polymer particles per unit volume in the reference solution.

More concretely, the powder formulation for tissue repair treatment of the present invention may be characterized in that, at dilution factor of 32, the number of polymer particles per unit volume in diluted solution is 65 % or higher, 70 % or higher, 75 % or higher, or 80 % or higher, based on 100 % of the number of polymer particles per unit volume in the reference solution.

In an embodiment, the powder formulation for tissue repair treatment of the present invention may be characterized in that, at dilution factor of 64, the number of polymer particles per unit volume in diluted solution is 40 % or higher (for example, 40 to 59 %), based on 100 % of the number of polymer particles per unit volume in the reference solution.

More concretely, the powder formulation for tissue repair treatment of the present invention may be characterized in that, at dilution factor of 64, the number of polymer particles per unit volume in diluted solution is 45 % or higher, 50 % or higher, or 55 % or higher, based on 100 % of the number of polymer particles per unit volume in the reference solution.

In an embodiment, the number of polymer particles per unit volume in solution can be measured by Flow Cytometer (FC). Flow Cytometer is a method wherein a solution containing polymer particles is irradiated with a laser while flowing at a specific flow rate for a specific time, and the number of events scattered into the detection area is counted and measured as the number of particles, and for example, it can be performed by using APOGEE equipment. In case of using APOGEE equipment, the number of particles having an average diameter of from 20 to 5000 nm can be measured. That is, according to an embodiment, the polymer particles contained in the powder formulation for tissue repair treatment of the present invention may be particles having an average diameter of from 20 to 5000 nm.

In an embodiment, the hydrophilic biocompatible polymer may be selected from the group consisting of polyethylene glycol or its derivative (e.g., alkoxy- or hydroxy-polyethylene glycol), polyvinylpyrrolidone, polyvinyl alcohol, polyacrylamide, and combination thereof, and more concretely, it may be selected from the group consisting of polyethylene glycol (PEG), methoxypolyethylene glycol (mPEG), and combination thereof.

In an embodiment, the hydrophobic biocompatible polymer may be polymer of alpha (α)-hydroxy acid-derived monomers, and more concretely, it may be selected from the group consisting of polylactide, polyglycolide, poly(lactic-glycolide), polymandelic acid, polycaprolactone, polydioxan-2-one, polyamino acid, polyorthoester, polyanhydride, polycarbonate and combination thereof, and still more concretely, it may be selected from the group consisting of polylactide, polyglycolide, poly(lactic-glycolide) and combination thereof.

Concretely, the number average molecular weight (unit: g/mol) by GPC of the hydrophilic biocompatible polymer (Mn1) may be 1,000 or more, 2,000 or more, 3,000 or more, or 4,000 or more, and also it may be 30,000 or less, 25,000 or less, 20,000 or less, 19,000 or less, 18,000 or less, 17,000 or less, 16,000 or less, or 15,000 or less.

Concretely, the number average molecular weight (unit: g/mol) by GPC of the hydrophobic biocompatible polymer (Mn2) may be 500 or more, 1,000 or more, 1,500 or more, or 2,000 or more, and also it may be 30,000 or less, 25,000 or less, 20,000 or less, 19,000 or less, 18,000 or less, 17,000 or less, 16,000 or less, or 15,000 or less.

Concretely, the total number average molecular weight by GPC of the biocompatible copolymer may be 2,000 or more, 5,000 or more, 8,000 or more, or 10,000 or more, and also it may be 40,000 or less, 35,000 or less, 30,000 or less, or 25,000 or less.

In an embodiment, in the biocompatible copolymer, the ratio (Mn1/Mn2) of the number average molecular weight (Mn1) of the hydrophilic biocompatible polymer to the number average molecular weight (Mn2) of the hydrophobic biocompatible polymer may be 2.5 or less.

The number average molecular weights of the hydrophilic and hydrophobic biocompatible polymers may be measured, for example, by gel permeation chromatography (GPC).

Gel permeation chromatography (GPC) is a method wherein the physical elution mechanism is that large components are eluted first and small components are eluted later depending on the size of the hydrodynamic volume of the analyzed components, and thus large molecules cannot enter the pores of the porous gel and pass through quickly while small molecules can enter the pores of the gel and be retained therein and thus pass through slowly, and the relative molecular weights are analyzed in the order of molecules passing through the column more quickly.

More concretely, the Mn1/Mn2 ratio in the biocompatible copolymer may be 2.5 or less, 2.3 or less, 2.1 or less, 2.0 or less, or 1.8 or less. There is no special limitation to the lower limit of the Mn1/Mn2 ratio, and for example, it may be 0.5 or more, 0.6 or more, 0.7 or more, 0.8 or more, or 0.9 or more, but it is not limited thereto.

In addition to the biocompatible copolymer explained above, the powder formulation for tissue repair treatment of the present invention may further comprise one or more additives that can be conventionally used in pharmaceutical powder formulation.

In an embodiment, the powder formulation for tissue repair treatment of the present invention may further comprise freeze-drying aid (also called freeze-drying agent).

Concretely, the freeze-drying aid may be one or more selected from the group consisting of lactose, maltose, sucrose, trehalose, mannitol, sorbitol, maltitol, xylitol, and lactitol.

In an embodiment, the powder formulation for tissue repair treatment of the present invention may further comprise local anesthetic.

Concretely, the local anesthetic may be one or more selected from the group consisting of ambucaine, amolanone, amylocaine, benoxinate, benzocaine, betoxycaine, biphenamine, bupivacaine, butacaine, butamben, butanilicaine, butethamine, butoxycaine, carticaine, chloroprocaine, cocaethylene, cocaine, cyclomethycaine, dibucaine, dimethysoquin, dimethocaine, diperodon, dicyclonine, ecgonidine, ecgonine, ethyl chloride, etidocaine, beta-eucaine, euprocin, fenalcomine, formocaine, hexylcaine, hydroxytetracaine, isobutyl p-aminobenzoate, leucinocaine mesylate, levoxadrol, lidocaine, mepivacaine, meprylcaine, metabutoxycaine, methyl chloride, myrtecaine, naepaine, octacaine, orthocaine, oxethazaine, parethoxycaine, phenacaine, phenol, piperocaine, piridocaine, polidocanol, pramoxine, prilocaine, procaine, propanocaine, proparacaine, propipocaine, propoxycaine, pseudococaine, pyrrocaine, ropivacaine, salicyl alcohol, tetracaine, tolycaine, trimecaine, zolamine, and salts thereof.

The powder formulation for tissue repair treatment of the present invention may be a formulation dried by freeze-drying or other drying method (e.g., spin drying, etc.) and thus having a powder status.

In an embodiment, the powder formulation for tissue repair treatment of the present invention may be prepared by a method comprising the steps of: polymerizing monomers for the above-explained hydrophobic biocompatible polymer in the presence of the above-explained hydrophilic biocompatible polymer to prepare a copolymer of hydrophilic biocompatible polymer and a hydrophobic biocompatible polymer; and drying the prepared copolymer, but it is not limited thereto.

The step of polymerizing monomers for the hydrophobic biocompatible polymer in the presence of the hydrophilic biocompatible polymer can be conducted according to known method and condition.

Concretely, the drying of the biocompatible copolymer can be conducted by freeze-drying or other drying method (e.g., spin drying, etc.), and more concretely it can be conducted by freeze-drying, but it is not limited thereto. The freeze-drying of the biocompatible copolymer can be conducted in the presence of freeze-drying aid as explained above, but it is not limited thereto.

The powder formulation for tissue repair treatment of the present invention is easily dissolved in aqueous medium at room temperature to form polymer particles (for example, particles exhibiting an average diameter in the range of from 20 nm to 5000 nm), and after being introduced into the body, the particles undergo self-assembly through hydrophobic aggregation of hydrophobic polymer under the influence of the environment in the body to form a large structure, which is not phagocytosed by macrophages and induces collagen, resulting in exhibition of excellent tissue repair effect.

Without being bound to theory, the aforesaid characteristics of the powder formulation for tissue repair treatment of the present invention (i.e., when a reference solution containing the powder formulation at a concentration of 7.5 % by weight is diluted according to a series of subsequently increasing dilution factors, (i) in a dilution factor range of from 4 to 8, the number of polymer particles per unit volume in diluted solution is the same as or higher than the number of polymer particles per unit volume in the reference solution; and (ii) in a dilution factor range of 8 or higher, the number of polymer particles per unit volume in diluted solution decreases as the dilution factor increases) may be considered to be due to such a feature of self-assembly of polymer particles. That is, in the powder formulation for tissue repair treatment of the present invention, it is considered that, when prepared as a solution with a concentration of the reference solution or higher, the polymer particles in the solution are self-assembled, and as the solution is diluted, in a dilution factor range of from 4 to 8, the self-assembled particle clusters are divided and the number of particles per unit volume of the solution increases or remains the same despite dilution, and if dilution continues in a dilution factor range of 8 or higher, there will no longer be particle clusters to be divided, and as dilution progresses, the number of particles per unit volume of the solution decreases. To the contrary, if the polymer particles in the solution are not self-assembled, the number of particles per unit volume of the solution consistently decreases as dilution progresses.

In the present invention, the tissue repair effect refers to the effect of returning the tissue to its original state when necrosis, defects, etc. occur in skin tissue due to trauma, inflammation, aging, etc.

Therefore, the other aspect of the present invention provides an injection composition for tissue repair treatment, comprising the powder formulation for tissue repair treatment of the present invention; and a pharmaceutically acceptable carrier for injection.

As the pharmaceutically acceptable carrier for injection comprised in the injection composition for tissue repair treatment of the present invention, conventional one can be used without limitation, and for example, it may be selected from the group consisting of a solution containing hyaluronic acid (hyaluronic acid solution), distilled water for injection, physiological saline, 5% glucose, buffer solutions (e.g., phosphate buffer solution (PBS)), and combination thereof, but it is not limited thereto.

In addition to the components explained above, the injection composition for tissue repair treatment of the present invention may further comprise one or more conventional additives that can be used in injection formulation.

Another aspect of the present invention provides a method for preparing an injection composition for tissue repair treatment, the method comprising: mixing the powder formulation for tissue repair treatment of the present invention and a pharmaceutically acceptable carrier for injection at room temperature.

In the method for preparing an injection composition for tissue repair treatment of the present invention, "room temperature" means 1 to 30°C, 20 to 30°C, 22 to 28°C, more specifically 24 to 26°C (e.g., 25°C).

The powder formulation of the existing tissue repair polymer product must be heated (e.g., the temperature must be raised between the melting point of the polymer and the boiling point of water) to be manufactured in the form of an aqueous solution. However, the powder formulation for tissue repair treatment of the present invention is easily dissolved in aqueous medium even at room temperature, and thus its injectable composition in the form of an aqueous solution can be easily prepared and used at room temperature.

The present invention will be explained below in more detail with reference to the following Examples. However, the Examples are only to illustrate the invention, and the scope of the present invention is not limited thereby in any manner.

### EXAMPLES

### Preparation Example 1: Preparation of powder formulation

In order to obtain a biocompatible copolymer with a target number average molecular weight (Mn) of 15,000 g/mol, in the presence of methoxypolyethyleneglycol (mPEG) with a number average molecular weight (Mn1) of 9,850 g/mol (measured by GPC) as a hydrophilic biocompatible polymer, D,L-lactide monomer was polymerized in the presence of a catalyst, and then the resulting copolymer was dried to obtain a powder formulation. As a result of GPC measurement, the number average molecular weight (Mn) of the prepared mPEG-Poly(D,L-lactide) copolymer was 15,500 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 1.74.

### Preparation Example 2: Preparation of powder formulation

Excepting that the target number average molecular weight (Mn) of the biocompatible copolymer was set to 17,000 g/mol, a powder formulation was prepared in the same manner as Preparation Example 1. As a result of GPC measurement, the number average molecular weight (Mn) of the prepared mPEG-Poly(D,L-lactide) copolymer was 17,200 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 1.34.

### Preparation Example 3: Preparation of powder formulation

Excepting that the target number average molecular weight (Mn) of the biocompatible copolymer was set to 20,000 g/mol, a powder formulation was prepared in the same manner as Preparation Example 1. As a result of GPC measurement, the number average molecular weight (Mn) of the prepared mPEG-Poly(D,L-lactide) copolymer was 20,200 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 0.95.

### Preparation Example 4: Preparation of powder formulation

In order to obtain a biocompatible copolymer with a target number average molecular weight (Mn) of 30,000 g/mol, in the presence of methoxypolyethyleneglycol (mPEG) with a number average molecular weight (Mn1) of 20,000 g/mol (measured by GPC) as a hydrophilic biocompatible polymer, D,L-lactide monomer was polymerized in the presence of a catalyst, and then the resulting copolymer was dried to obtain a powder formulation. As a result of GPC measurement, the number average molecular weight (Mn) of the prepared mPEG-Poly(D,L-lactide) copolymer was 29,550 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 2.1.

### Comparative Example 1

A formulation prepared as powder by freeze-drying a commercial product consisting of mPEG-Polycaprolactone copolymer (Miracle L, Dexlevo Co., Ltd.) was used as Comparative Example 1.

When the mPEG-Polycaprolactone powder formulation of Comparative Example 1 was prepared as an aqueous solution by mixing at room temperature, particle size analysis was difficult since the polymer particles were precipitated due to poor dispersion stability, and the reliability of the results was low. Therefore, water was added to the mPEG-Polycaprolactone powder formulation, and the mixture was heated to 80°C and then mixed to prepare an aqueous polymer colloidal solution, and analysis was conducted thereto.

### Comparative Example 2: Preparation of powder formulation

Excepting that the target number average molecular weight (Mn) of the biocompatible copolymer was set to 12,000 g/mol, a powder formulation was prepared in the same manner as Preparation Example 1. As a result of GPC measurement, the number average molecular weight (Mn) of the prepared mPEG-Poly(D,L-lactide) copolymer was 12,350 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 2.8.

### Comparative Example 3: Preparation of powder formulation

In order to obtain a biocompatible copolymer with a target number average molecular weight (Mn) of 25,000 g/mol, in the presence of methoxypolyethyleneglycol (mPEG) with a number average molecular weight (Mn1) of 20,000 g/mol (measured by GPC) as a hydrophilic biocompatible polymer, D,L-lactide monomer was polymerized in the presence of a catalyst, and then the resulting copolymer was dried to obtain a powder formulation. As a result of GPC measurement, the number average molecular weight (Mn) of the prepared mPEG-Poly(D,L-lactide) copolymer was 25,650 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 3.5.

### Comparative Example 4: Preparation of powder formulation

In order to obtain a biocompatible copolymer with a target number average molecular weight (Mn) of 23,000 g/mol, in the presence of methoxypolyethyleneglycol (mPEG) with a number average molecular weight (Mn1) of 20,000 g/mol (measured by GPC) as a hydrophilic biocompatible polymer, D,L-lactide monomer was polymerized in the presence of a catalyst, and then the resulting copolymer was dried to obtain a powder formulation. As a result of GPC measurement, the number average molecular weight (Mn) of the prepared mPEG-Poly(D,L-lactide) copolymer was 23,500 g/mol, and the hydrophobic polymer molecular weight was calculated by subtracting the hydrophilic polymer molecular weight from the measured molecular weight of the copolymer. The ratio (Mn1/Mn2) of the number average molecular weight (Mn1) of mPEG (hydrophilic polymer in the copolymer) to the number average molecular weight (Mn2) of Poly(D,L-lactide) (hydrophobic polymer in the copolymer) was 5.7.

### Comparative Example 5: Powder formulation of PCL nanoparticles

Polycaprolactone (PCL) nanoparticles (product number PCL200-5ML-A) with an average particle diameter of 200 nm were purchased from Sigma-Aldrich and freeze-dried to obtain a powder formulation.

### Experimental Example 1: Analysis of polymer molecular weight

The number average molecular weights (Mn) of the polymers of Preparation Examples 1 to 4 and Comparative Examples 1 to 5 were measured by performing Gel Permeation Chromatography (GPC) under the conditions shown in the following table. The results are shown in Table 1 below.

| | |
|---|---|
| Instrument | Agilent Infinity 1260 GPC/SEC system |
| Column | One Mixed-C guard column and two Mixed-C analytical columns |
| Eluent | Chloroform (HPLC grade) |
| Detector | Refractive Index (RI) Detector |
| Flow rate | 1.0 mL/min |
| Temperature | 35°C |
| Concentration | 0.1~ 0.2 w/v % |
| Injection volume | 100 µL |
| Standard material | 9 kinds of PS (195300/69650/30230/19500/12980/6320/3090/1290/945g/mol) |

### Experimental Example 2: Measurement of the number of polymer particles in solution according to aqueous medium dilution of powder formulation

Each of the formulations of Preparation Examples 1 to 4 and Comparative Examples 1 to 5 was added to water for injection as aqueous medium to prepare a solution with concentration of 7.5 % by weight (reference solution), and the number of particles with an average diameter of 20 to 5000 nm present in a unit volume of the solution was measured by using APOGEE equipment (model name: A60 Micro Plus; manufacturer: APOGEE flow systems) according to flow cytometry (FC). The measurement conditions were as follows:
(1) Equipment conditions and parameter setting
   1) Laser source: 405 nm (Violet) / 70mW
   2) Flow rate: 0.75 µl/min
   3) Injection volume: 100 µl
   4) PMT: SALS: 415V / MALS: 400V / LALS: 400V (STD: set to about 40,000ets/µl)
   5) Acquisition time: 120s
   6) Thresholds: SALS-5, MALS-13, LALS-3
(2) APOGEE STD measurement
   Standard material: Cat #1527, APOGEE Mix 25 ml (PS80, 110, 500 & SI80, 240, 30, 590, 880, 1300nm)
   Lot: CAL0145
(3) Measurement and conversion of number of events

The number of events within the same ROD area was measured, and if the measurement time was less than 120 s, the number of events was determined by converting the measured result to that of the measurement up to 120 s.

Thereafter, the reference solution was diluted with water for injection according to dilution factors of 2, 4, 8, 16, 32, and 64, and for each diluted solution, the number of particles with an average diameter of 20 to 5000 nm present in the unit volume of the solution was measured in the same manner.

The number of events measured or converted after measurement is shown as the number of particles in Tables 2 and 3 below.

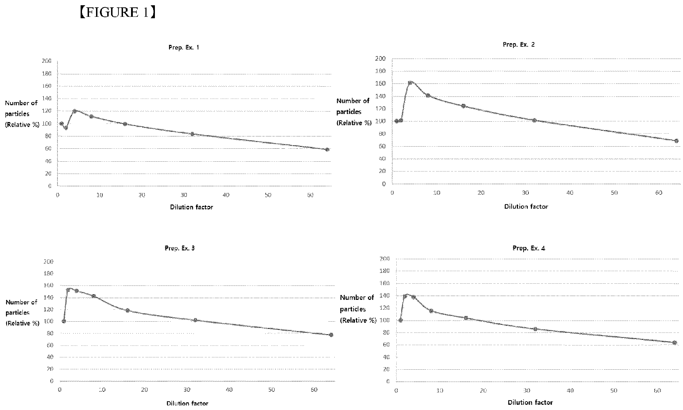

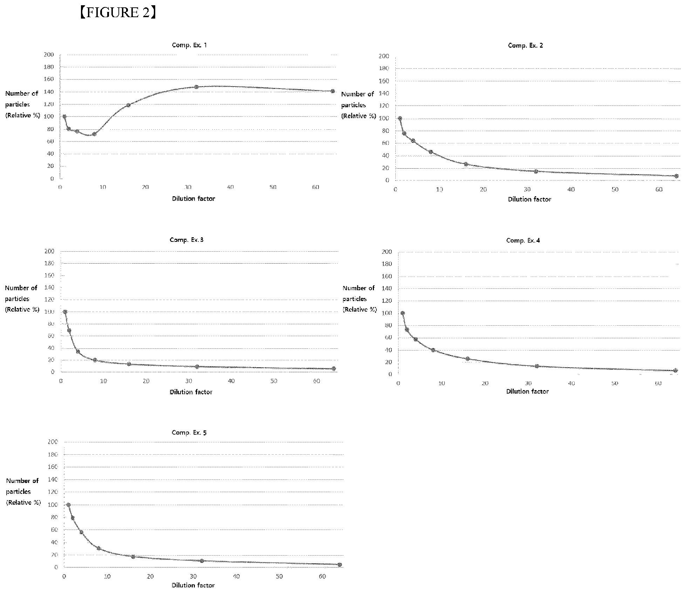

In addition, the number of particles in the diluted solution is expressed as a relative %, based on 100 % of the number of particles in the reference solution, and is shown in Tables 2 and 3 below, and the relative % change in the number of particles in the diluted solution according to the increase in dilution factor is shown in Figures 1 and 2.

**[Table 1]**

| | Hydrophilic polymer M.W. (Mn1) | Hydrophobic polymer M.W. (Mn2) | Copolymer M.W. | Mn1/Mn2 ratio |
|---|---|---|---|---|
| Prep. Ex. 1 | 9,850 | 5,650 | 15,500 | 1.74 |
| Prep. Ex. 2 | 9,850 | 7,350 | 17,200 | 1.34 |
| Prep. Ex. 3 | 9,850 | 10,350 | 20,200 | 0.95 |
| Prep. Ex. 4 | 20,000 | 9,550 | 29,550 | 2.1 |
| Comp. Ex. 1 (Miracle L) | - | - | 25,300 | - |
| Comp. Ex. 2 | 9,850 | 3,500 | 12,350 | 2.8 |
| Comp. Ex. 3 | 20,000 | 5,650 | 25,650 | 3.5 |
| Comp. Ex. 4 | 20,000 | 3,500 | 23,500 | 5.7 |
| Comp. Ex. 5 (PCL nanoparticle) | - | 10,000 | 10,000 | - |

| | | | | |
|---|---|---|---|---|
| (Prep. Ex.: Preparation Example; Comp. Ex.: Comparative Example; M.W.: Molecular weight) | | | | |

**[Table 2]**

| | Dilution factor | Acquisition time | Number of particles (raw data) | Number of particles (converted to 120s) | Relative number of particles (%) |
|---|---|---|---|---|---|
| Prep. Ex. 1 | 1 (Reference solution) | 104 | 4,885,643 | 5,637,280 | 100 |
| | 2 | 112 | 4,902,089 | 5,252,238 | 93.2 |
| | 4 | 87 | 4,879,556 | 6,730,422 | 119.4 |
| | 8 | 93 | 4,874,551 | 6,289,743 | 111.6 |
| | 16 | 105 | 4,899,322 | 5,599,225 | 99.3 |
| | 32 | 120 | 4,696,184 | 4,696,184 | 83.3 |
| | 64 | 120 | 3,296,434 | 3,296,434 | 58.5 |
| | PBS Buffer (Control) | 120 | 2,981 | 2,981 | - |
| Prep. Ex. 2 | 1 (Reference solution) | 120 | 3,178,179 | 3,178,179 | 100 |
| | 2 | 105 | 2,826,675 | 3,230,486 | 101.6 |
| | 4 | 113 | 4,821,768 | 5,120,462 | 161.1 |
| | 8 | 120 | 4,482,536 | 4,482,536 | 141.0 |
| | 16 | 120 | 3,941,403 | 3,941,403 | 124.0 |
| | 32 | 120 | 3,223,131 | 3,223,131 | 101.4 |
| | 64 | 120 | 2,187,998 | 2,187,998 | 68.8 |
| | PBS Buffer (Control) | 120 | 5,775 | 5,775 | - |
| Prep. Ex. 3 | 1 (Reference solution) | 120 | 3,372,791 | 3,372,791 | 100 |
| | 2 | 112 | 4,809,407 | 5,152,936 | 152.8 |
| | 4 | 114 | 4,828,264 | 5,082,383 | 150.7 |
| | 8 | 120 | 4,796,730 | 4,796,730 | 142.2 |
| | 16 | 120 | 3,979,788 | 3,979,788 | 118.0 |
| | 32 | 120 | 3,434,348 | 3,434,348 | 101.8 |
| | 64 | 120 | 2,595,191 | 2,595,191 | 76.9 |
| | PBS Buffer (Control) | 120 | 4,120 | 4,120 | - |
| Prep. Ex. 4 | 1 (Reference solution) | 120 | 3,893,081 | 3,893,081 | 100 |
| | 2 | 106 | 4,775,705 | 5,406,458 | 138.9 |
| | 4 | 107 | 4,776,381 | 5,356,689 | 137.6 |
| | 8 | 120 | 4,495,529 | 4,495,529 | 115.5 |
| | 16 | 120 | 4,019,514 | 4,019,514 | 103.2 |
| | 32 | 120 | 3,330,238 | 3,330,238 | 85.5 |
| | 64 | 120 | 2,466,658 | 2,466,658 | 63.4 |
| | PBS Buffer (Control) | 120 | 3,080 | 3,080 | - |

| | | | | | |
|---|---|---|---|---|---|
| (Prep. Ex.: Preparation Example) | | | | | |

**[Table 3]**

| | Dilution factor | Acquisition time | Number of particles (raw data) | Number of particles (converted to 120s) | Relative number of particles (%) |
|---|---|---|---|---|---|
| Comp. Ex. 1 (Miracle L) | 1 (Reference solution) | 53 | 4,610,374 | 10,438,583 | 100 |
| | 2 | 66 | 4,627,279 | 8,413,235 | 80.6 |
| | 4 | 69 | 4,572,768 | 7,952,640 | 76.2 |
| | 8 | 70 | 4,387,663 | 7,521,708 | 72.1 |
| | 16 | 44 | 4,529,060 | 12,351,982 | 118.3 |
| | 32 | 37 | 4,758,783 | 15,433,891 | 147.9 |
| | 64 | 39 | 4,793,502 | 14,749,237 | 141.3 |
| | PBS Buffer (Control) | 120 | 26,525 | 26,525 | - |
| Comp. Ex. 2 | 1 (Reference solution) | 86 | 5,726,776 | 7,990,850 | 100 |
| | 2 | 97 | 4,871,100 | 6,026,103 | 75.4 |
| | 4 | 120 | 5,086,804 | 5,086,804 | 63.7 |
| | 8 | 120 | 3,665,387 | 3,665,387 | 45.9 |
| | 16 | 120 | 2,094,915 | 2,094,915 | 26.2 |
| | 32 | 120 | 1,157,461 | 1,157,461 | 14.5 |
| | 64 | 120 | 571,284 | 571,284 | 7.1 |
| | PBS Buffer (Control) | 120 | 4,965 | 4,965 | - |
| Comp. Ex. 3 | 1 (Reference solution) | 86 | 3,845,621 | 5,365,983 | 100 |
| | 2 | 97 | 2,984,531 | 3,692,203 | 68.8 |
| | 4 | 120 | 1,846,321 | 1,846,321 | 34.4 |
| | 8 | 120 | 1,059,648 | 1,059,648 | 19.7 |
| | 16 | 120 | 701,358 | 701,358 | 13.1 |
| | 32 | 120 | 488,432 | 488,432 | 9.1 |
| | 64 | 120 | 294,513 | 294,513 | 5.5 |
| | PBS Buffer (Control) | 120 | 4,965 | 4,965 | - |

| | | | | | |
|---|---|---|---|---|---|
| (Comp. Ex.: Comparative Example) | | | | | |

**[Table 3] (continued)**

| | Dilution factor | Acquisition time | Number of particles (raw data) | Number of particles (converted to 120s) | Relative number of particles (%) |
|---|---|---|---|---|---|
| Comp. Ex. 4 | 1 (Reference solution) | 86 | 4,821,357 | 6,727,475 | 100 |
| | 2 | 97 | 3,984,531 | 4,929,317 | 73.3 |
| | 4 | 120 | 3,846,321 | 3,846,321 | 57.2 |
| | 8 | 120 | 2,687,458 | 2,687,458 | 39.9 |
| | 16 | 120 | 1,712,356 | 1,712,356 | 25.5 |
| | 32 | 120 | 887,266 | 887,266 | 13.2 |
| | 64 | 120 | 412,385 | 412,385 | 6.1 |
| | PBS Buffer (Control) | 120 | 4,965 | 4,965 | - |
| Comp. Ex. 5 (PCL nanoparticle) | 1 (Reference solution) | 94 | 4,993,310 | 6,374,438 | 100 |
| | 2 | 106 | 4,451,922 | 5,039,912 | 79.1 |
| | 4 | 107 | 3,196,016 | 3,584,317 | 56.2 |
| | 8 | 120 | 1,930,581 | 1,930,581 | 30.3 |
| | 16 | 120 | 1,101,409 | 1,101,409 | 17.3 |
| | 32 | 120 | 656,393 | 656,393 | 10.3 |
| | 64 | 120 | 282,928 | 282,928 | 4.4 |
| | PBS Buffer (Control) | 120 | 4,965 | 4,965 | - |

| | | | | | |
|---|---|---|---|---|---|
| (Comp. Ex.: Comparative Example) | | | | | |

### Experimental Example 3: Verification of efficacy as a formulation for tissue repair through animal tests

The efficacy for tissue repair of the powder formulation according to the present invention was verified through animal tests. The animal tests were conducted using 5-week-old SD rats (purchased from Orient Bio Co., Ltd.).

In the animal tests, saline and test materials were administered to each 5-week-old SD rat on both sides. During the test period, the rearing environment was set at a temperature of 24 ± 2°C, relative humidity of 50 ± 10%, and lighting time of 12 hours, and food was allowed to be eaten freely.

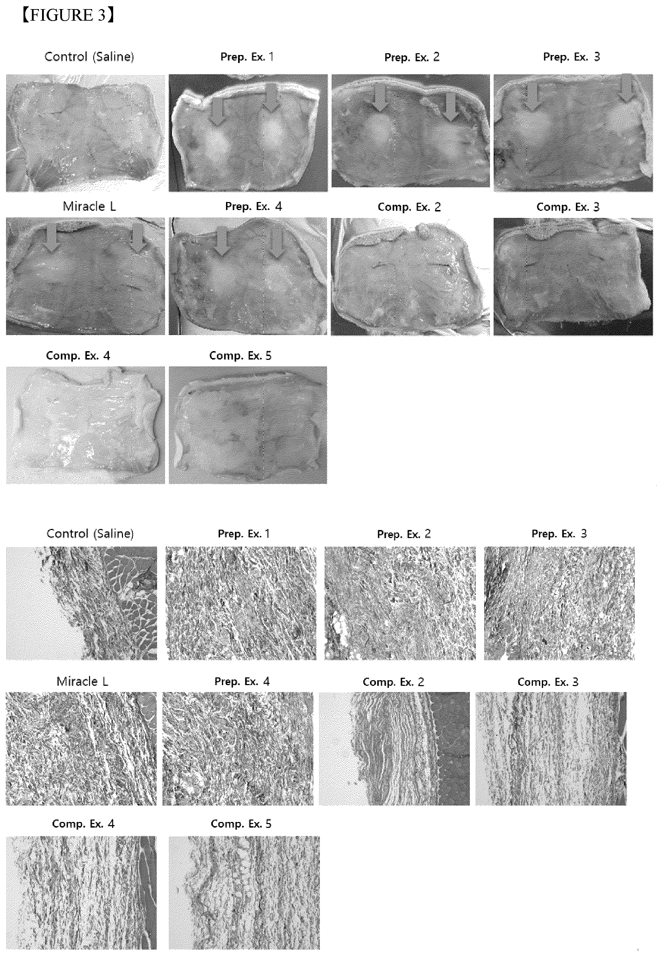

Physiological saline as Control, an aqueous solution of each powder formulation prepared in Preparation Examples 1 to 4 dissolved in water for injection (10% concentration) as a test material, and Comparative Example 1 product (Miracle L, prepared at 20% concentration) and an aqueous solution of each powder formulation of Comparative Examples 2 to 5 dissolved in water for injection (10% concentration) as comparison group were injected at a constant rate of 100 µl. After 6 weeks, the test animals were sacrificed, and the skin tissue at the sample injection site (the area indicated by the arrow in the upper part of Figure 3) was stained with Masson's Trichorme (MT) and collagen formation in the tissue was to observed in order to evaluate the ability of new collagen biosynthesis. The results are shown in Figure 3 (the lower part of Figure 3).

From Figure 3, it was confirmed that, as compared with Comparative Examples, especially Miracle L, the aqueous solutions of the formulations of Preparation Examples showed excellent collagen formation, and in particular, the formulation of Preparation Example 3 had a large number of formed collagen fibers which were significantly thicker than Comparative Examples, especially Miracle L.

## Claims

1. A powder formulation for tissue repair treatment, comprising a copolymer of hydrophilic biocompatible polymer and hydrophobic biocompatible polymer, wherein:
when a reference solution containing the powder formulation at a concentration of 7.5 % by weight is diluted according to a series of subsequently increasing dilution factors,
in a dilution factor range of from 4 to 8, the number of polymer particles per unit volume in diluted solution is the same as or higher than the number of polymer particles per unit volume in the reference solution; and
in a dilution factor range of 16 or higher, the number of polymer particles per unit volume in diluted solution decreases as the dilution factor increases.

2. The powder formulation for tissue repair treatment of Claim 1, wherein, in a dilution factor range of from 4 to 16, the number of polymer particles per unit volume in diluted solution is the same as or higher than the number of polymer particles per unit volume in the reference solution.

3. The powder formulation for tissue repair treatment of Claim 1, wherein, in a dilution factor range of from 4 to 32, the number of polymer particles per unit volume in diluted solution is the same as or higher than the number of polymer particles per unit volume in the reference solution.

4. The powder formulation for tissue repair treatment of Claim 1, wherein, in a dilution factor range of 8 or higher, the number of polymer particles per unit volume in diluted solution decreases as the dilution factor increases.

5. The powder formulation for tissue repair treatment of Claim 1, wherein, in a dilution factor range of 4 or higher, the number of polymer particles per unit volume in diluted solution decreases as the dilution factor increases.

6. The powder formulation for tissue repair treatment of Claim 1, wherein the hydrophilic biocompatible polymer is selected from the group consisting of polyethylene glycol or its derivative, polyvinylpyrrolidone, polyvinyl alcohol, polyacrylamide and combination thereof.

7. The powder formulation for tissue repair treatment of Claim 1, wherein the hydrophobic biocompatible polymer is selected from the group consisting of polylactide, polyglycolide, poly(lactic-glycolide), polymandelic acid, polycaprolactone, polydioxan-2-one, polyamino acid, polyorthoester, polyanhydride, polycarbonate and combination thereof.

8. The powder formulation for tissue repair treatment of Claim 1, wherein the hydrophilic biocompatible polymer is selected from the group consisting of polyethylene glycol (PEG), methoxypolyethylene glycol (mPEG) and combination thereof, and the hydrophobic biocompatible polymer is selected from the group consisting of polylactide, polyglycolide, poly(lactic-glycolide) and combination thereof.

9. The powder formulation for tissue repair treatment of Claim 1, wherein the number average molecular weight of the hydrophilic biocompatible polymer by gel permeation chromatography is from 1,000 g/mol to 30,000 g/mol.

10. The powder formulation for tissue repair treatment of Claim 1, wherein the number average molecular weight of the hydrophobic biocompatible polymer by gel permeation chromatography is from 500 g/mol to 30,000 g/mol.

11. The powder formulation for tissue repair treatment of Claim 1, wherein the number average molecular weight of the biocompatible copolymer by gel permeation chromatography is from 2,000 g/mol to 40,000 g/mol.

12. The powder formulation for tissue repair treatment of Claim 1, wherein, in the biocompatible copolymer, the ratio (Mn1/Mn2) of the number average molecular weight (Mn1) of the hydrophilic biocompatible polymer to the number average molecular weight (Mn2) of the hydrophobic biocompatible polymer is 2.5 or less.

13. The powder formulation for tissue repair treatment of Claim 1, further comprising freeze-drying aid.

14. The powder formulation for tissue repair treatment of Claim 1, further comprising local anesthetic.

15. An injection composition for tissue repair treatment, comprising:
the powder formulation for tissue repair treatment of any one of Claims 1 to 14; and
a pharmaceutically acceptable carrier for injection.

16. A method for preparing an injection composition for tissue repair treatment, the method comprising: mixing the powder formulation for tissue repair treatment of any one of Claims 1 to 14 and a pharmaceutically acceptable carrier for injection at room temperature.
